# EUROPEAN PATENT SPECIFICATION

(11) **EP 4 203 963 B1**
(45) Date of publication and mention of the grant of the patent: **21.01.2026**
(21) Application number: 21765720.4
(22) Date of filing: 30.08.2021
(51) Int. Cl.: A61K 31/519, A61K 31/5377, A61K 45/06, A61P 35/00

(54) **COMBINATION THERAPY OF NAPORAFENIB AND TRAMETINIB FOR USE IN THE TREATMENT OF SARCOMA**
KOMBINATIONSTHERAPIE MIT NAPORAFENIB UND TRAMETINIB ZUR VERWENDUNG BEI DER BEHANDLUNG VON SARKOM
POLYTHÉRAPIE COMPRENANT NAPORAFENIB ET TRAMETINIB POUR UNE UTILISATION DANS LE TRAITEMENT D'UN SARCOME

(30) Priority: 31.08.2020 US 202063072528 P
(43) Date of publication of application: 05.07.2023
(73) Proprietor: Novartis AG, 4056 Basel (CH); Board of Regents, The University of Texas System, Austin, TX 78701 (US)
(72) Inventor: CAPONIGRO, Giordano, Cambridge, Massachusetts 02139 (US); COOKE, Vesselina, Cambridge, Massachusetts 02139 (US); VASEVA, Angelina, San Antonio, Texas 78229 (US)
(74) Representative: Evans, Sophie Elizabeth
(86) International application number: PCT/IB2021/057904
(87) International publication number: WO 2022/043955

(56) References cited:
- WO-A1-2018/203219
- WO-A1-2019/051296
- RASSIDAKIS GEORGE Z ET AL: "Trametinib and Dabrafenib in histiocytic sarcoma transdifferentiated from chronic lymphocytic leukemia with aand a uniquemutation", ANNALS OF HEMATOLOGY, BERLIN, DE, vol. 99, no. 3, 3 February 2020 (2020-02-03), pages 649 - 651, XP037054800, ISSN: 0939-5555, [retrieved on 20200203], DOI: 10.1007/S00277-020-03941-7
- LIU HAOTIAN ET AL: "BRAF mutation and its inhibitors in sarcoma treatment", CANCER MEDICINE, vol. 9, no. 14, 31 May 2020 (2020-05-31), GB, pages 4881 - 4896, XP055853555, ISSN: 2045-7634, DOI: 10.1002/cam4.3103
- WATANABE SHO ET AL: "V600E mutation is a potential therapeutic target for a small subset of synovial sarcoma", MODERN PATHOLOGY, NATURE PUBLISHING GROUP, GB, vol. 33, no. 9, April 2020 (2020-04-01), pages 1660 - 1668, XP037230162, ISSN: 0893-3952, [retrieved on 20200401], DOI: 10.1038/S41379-020-0530-3
- STUART DARRIN D ET AL: "Pharmacological profile and anti-tumor properties of LXH254, a highly selective RAF kinase inhibitor", CANCER RESEARCH, vol. 78, no. 13 suppl, July 2018 (2018-07-01), pages DDT01 - 04, XP055853496, DOI: 10.1158/1538-7445.AM2018-DDT01-04
- RAMURTHY SAVITHRI ET AL: "Design and Discovery of N -(3-(2-(2-Hydroxyethoxy)-6-morpholinopyridin-4-yl)-4-methylphenyl)-2-(trifluoromethyl)isonicotinamide, a Selective, Efficacious, and Well-Tolerated RAF Inhibitor Targeting RAS Mutant Cancers: The Path to the Clinic", JOURNAL OF MEDICINAL CHEMISTRY, vol. 63, no. 5, 6 May 2019 (2019-05-06), US, pages 2013 - 2027, XP055804283, ISSN: 0022-2623, DOI: 10.1021/acs.jmedchem.9b00161

## Description

### FIELD OF THE INVENTION

This invention provides a pharmaceutical combination comprising, or consisting of: (a) a Raf inhibitor, for example, a Compound of formula (I), as defined herein (also named Compound A or LXH254 hereinafter), or a pharmaceutically acceptable salt thereof, and (b) trametinib, or a pharmaceutically acceptable salt or solvate (for example the dimethyl sulfoxide solvate) thereof, for use in the treatment of sarcoma, especially RAS-mutant sarcoma. The invention also provides such a pharmaceutical combination for use in the treatment of rhabdomyosarcoma, most especially of embryonal rhabdomyosarcoma (ERMS). These and related invention embodiments are described in more detail below.

### BACKGROUND

The RAS/RAF/MEK/ERK or MAPK pathway is a key signaling cascade that drives cell proliferation, differentiation, and survival. Dysregulation of this pathway underlies many instances of tumorigenesis. Aberrant signaling or inappropriate activation of the MAPK pathway has been shown in multiple tumor types, including melanoma, lung and pancreatic cancer, and can occur through several distinct mechanisms, including activating mutations in RAS and BRAF. RAS is a superfamily of GTPases, and includes KRAS (v-Ki-ras2 Kirsten rat sarcoma viral oncogene homolog), which is a regulated signaling protein that can be turned on (activated) by various single-point mutations, which are known as gain of function mutations. The MAPK pathway is frequently mutated in human cancer with *KRAS* mutations being among the most frequent (approximately 30%). RAS mutations, particularly gain of function mutations, have been detected in 9-30% of all cancers, with *KRAS* mutations having the highest prevalence (86%), followed by *NRAS* (11%), and, infrequently, *HRAS* (3%) (Cox AD, Fesik SW, Kimmelman AC, et al (2014), Nat Rev Drug Discov. Nov; 13(11):828-51.). Targeting downstream RAS effector signaling, particularly the RAF-MEK-ERK mitogen-activated protein kinase cascade, is being intensively pursued as a therapeutic target for RAS-driven cancers. However, clinical success has been limited due to the emergence of multiple mechanisms of resistance. Moreover, despite significant recent progress, it is becoming evident that mechanisms of resistance and design of successful therapeutic strategies are highly context-dependent (i.e. cancer tissue origin, RAS isoform, mutation position) and require careful evaluation in each specific RAS-driven cancer type.

Rhabdomyosarcoma (RMS), an aggressive and highly malignant form of cancer, is the most common soft tissue sarcoma in children developing from striated skeletal muscle cells that have failed to fully differentiate. The two major subtypes, alveolar (ARMS) and embryonal (ERMS), differ in their histological features, genetic mutations and age of onset (Egas-Bejar D, Huh WW., Adolescent health, medicine and therapeutics 2014;5:115-25). While the 5-year survival for localized rhabdomyosarcoma is over 70%, it is only 30% for patients presenting with metastatic disease (Hettmer S et al., Rhabdomyosarcoma: current challenges and their implications for developing therapies. Cold Spring Harbor perspectives in medicine. 2014;4(11):a025650). The current standard treatments are cytotoxic therapies such as chemotherapy and radiation. Even when therapy is curative, the affected children face profound lifelong therapy-related complications and development of second malignancies (Punyko JA et al., Pediatric blood & cancer. 2005;44(7):643-53). Hopes for the future lay on identification of novel less toxic therapies such as immuno-therapies or intervention with drugs specific for cancer-driving cellular and molecular processes.

Sarcomas are rare tumours of connective tissue characterised by marked heterogeneity and including more than 70 histological subtypes, among them soft tissue sarcomas, like undifferentiated pleomorphic sarcoma (UPS) and rhabdomyosarcoma. Sarcomas represent 6-15% of pediatric cancers (<15 years), 11% of adolescent and young adult cancers (15-29 years), and 1-2% of adult cancers worldwide. In children aged 0-15 years, the largest group of soft tissue sarcomas consists of rhabdomyosarcoma. This occurs mainly in children younger than 7 years, with another incidence peak during adolescence (16-19 years). Prognosis of sarcomas varies with age, being substantially superior in children compared with young adults; the reasons for this discrepancy are not fully clear, however, they are multifactorial. Rhabdomyosarcomas show a high grade of malignancy and a marked propensity to metastasise, such that all patients with rhabdomyosarcoma are assumed to have micrometastatic disease at diagnosis, therefore requiring to be treated with systemic therapy. Rhabdomyosarcomas are generally characterised as having a good response to chemotherapy, with responses of roughly 80-90%, as well as good responses to radiotherapy in general. The backbone of treatment for pediatric patients with localised rhabdomyosarcoma so far is an intensive alkylation-based multidrug chemotherapy regimen given for 6-9 months. This and other treatment options are discussed in Winette T A van der Graaf et al., The Lancet Oncology, 2017 18(3), e166-e175.

This paper also points out that the few published series on adult patients described poor outcomes, with overall survival in the range of 20-50%. Some findings suggest that adult patients would fare better if they were treated with properly administered padiatric regimens.

With regard to pediatric rhabdomyosarcoma, also Daniel S. Rheee et al., J. of Pediatric Surgery 2020, https://doi.org/10.1016/j.jpedsurg.2020.06.015, provide a comprehensive review of the current literature and the most recent Children's Oncology Group studies and several international collaboratives, regarding surgical and medical treatment. Rassidakis G et al (Annals of Hematology, 2020, 99(3): 649-651) concerns the use of the MEK inhibitor trametinib and the RAF inhibitor dabrafenib for the treatment of histiocytic sarcoma. Liu H et al (Cancer Medicine, 2020, 9(14): 4881-4896) discloses the use of BRAF inhibitors in sarcoma treatment. It teaches that combinations of BRAF inhibitors and trametinib have already been used in the treatment of sarcoma. Watanabe S et al (Modern Pathology, 2020, 33(9): 1660-1668) discloses a combination of dabrafenib and trametinib for the treatment of a patient with intrathoracic synovial sarcoma harboring the BRAFV600E mutation.

Prognosis in rhabdomyosarcoma patients has been shown to be dependent on age, tumor site, resectability of tumor, tumor size, regional lymph node involvement, presence of metastasis, site and extent of metastasis, and biological and histopathological characterristics of the tumor cells. Survival after recurrence is poor, and new primary and salvage therapy strategies are needed.

Treatment of RMS, and in particular ERMS, therefore remains a high unmet medical need. There remains a need to provide effective treatment which is well tolerated, delays the onset of resistance, and also provides limited treatment toxicity such as skin rash and other side effects.

SUMMARY OF THE INVENTION The present invention is defined in the appended claims. Any embodiments which do not fall under the scope of the claims are not the subject of the present invention.

The references to the methods of treatment of this description are to be interpreted as references to compounds, pharmaceutical compositions and medicaments of the present invention for use in a method for treatment of the human (or animal) body by therapy.

It was found that a treatment combining the use of Compound A and trametinib leads to unexpectedly positive and even synergistic effects in the treatment of sarcoma, especially rhabdomyosarcoma, including embyronal rhabdomyosarcoma. The addition of trametinib to Compound A in a model of rhabdomyosarcoma also helped to overcome resistance by the tumor to treatment. The administration of the combination of Compound A and trametinib was also found to be well tolerated in mice models.

The present invention therefore provides a pharmaceutical combination comprising (or especially consisting of): (a) a Raf inhibitor such as a Compound of formula (I), as defined herein, or a pharmaceutically acceptable salt thereof, and trametinib, or a pharmaceutically acceptable salt or solvate thereof; with or without the presence of one or more pharmaceutically acceptable carrier materials; said combination especially for use in a method of (therapeutic) treatment of a sarcoma, especially rhabdomyosarcoma, more especially of embryonal rhabdomyosarcoma (ERMS).

This invention also provides the following embodiments:
- a pharmaceutical combination comprising (or especially consisting of) (a) a Raf inhibitor selected from a Compound of formula (I), as defined herein, or a pharmaceutically acceptable salt thereof, and trametinib, or a pharmaceutically acceptable salt or solvate, (with or without one or more pharmaceutically acceptable carrier materials) for use in the treatment of a sarcoma, especially rhabdomyosarcoma, more especially of embryonal rhabdomyosarcoma (ERMS);
- a method of treating sarcoma, in particular rhabdomyosarcoma, more especially of embryonal rhabdomyosarcoma (ERMS), especially in a patient, especially a pediatric patient, in need thereof, comprising administering to said patient a jointly therapeutically effective amount of said combination;
- the use of said a combination for the treatment of a sarcoma, in particular rhabdomyosarcoma, more especially of embryonal rhabdomyosarcoma (ERMS);
- a pharmaceutical composition comprising said combination and/or commercial packages thereto, especially for use in the treatment of a sarcoma, especially rhabdomyosarcoma, more especially of embryonal rhabdomyosarcoma (ERMS);
- a Raf inhibitor such as a Compound of the formula (I), or a pharmaceutically acceptable salt thereof, for use in a combination therapy with trametinib, or a pharmaceutically acceptable salt or solvate thereof; with or without one or more pharmaceutically acceptable carrier materials; in the treatment of sarcoma, especially rhabdomyosarcoma, more especially of embryonal rhabdomyosarcoma (ERMS);
- trametinib, or a pharmaceutically acceptable salt or solvate thereof, for use in a combination therapy with a Raf inhibitor such as a Compound of the formula (I), or a pharmaceutically acceptable salt thereof; with or without one or more pharmaceutically acceptable carrier materials; in the treatment of sarcoma, especially rhabdomyosarcoma, more especially of embryonal rhabdomyosarcoma (ERMS).

This invention also provides the Compound of formula (I), as defined herein, or a pharmaceutically acceptable salt thereof, for use in a combination therapy of sarcoma, especially rhabdomyosarcoma, more especially of embryonal rhabdomyosarcoma (ERMS), with trametinib, or a pharmaceutically acceptable salt or solvate thereof (preferably as the only other active ingredient).

### DETAILED DESCRIPTION OF THE INVENTION

The term "sarcoma" especially refers to a malignant tumor, a type of cancer that arises from transformed cells of mesenchymal (connective tissue) origin. Connective tissue is a broad term that includes bone , cartilage, fat, vascular, or hematopoietic tissues, and sarcomas can arise in any of these types of tissues. As a result, there are many subtypes of sarcoma, which are classified based on the specific tissue and type of cell from which the tumor originates. Sarcomas are *primary* connective tissue tumors, meaning that they arise in connective tissues.

A more preferred sarcoma amenable to treatment according to the present invention is myosarcoma, which may be a leiomyosarcoma (sarcoma of the smooth muscle) or especially rhabdomyosarcoma which is sarcoma of striated muscle or its ontogenic precursor tissues or cells.

The preferred sarcoma amenable to treatment according to the present invention is rhabdomyosarcoma, a term which includes embryonal (ERMS); or further alveolar (ARMS), anaplastic or pleomorphic, or spindle cell/sclerosing rhabdomyosarcoma. The present combination is particularly useful for treating ERMS.

A Raf inhibitor useful in the present combination is a Compound of the formula (I) 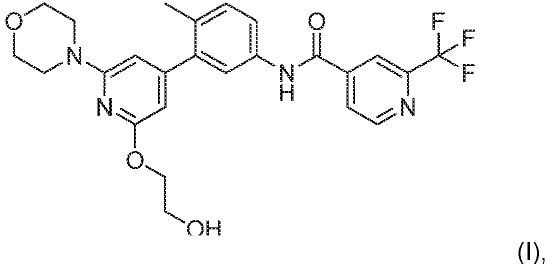 or a pharmaceutically acceptable salt thereof.

The compound of formula (I) is also known by the name N-(3-(2-(2-hydroxyethoxy)-6-morpholinopyridin-4-yl)-4-methylphenyl)-2-(trifluoromethyl)isonicotinamide, and also referred to herein as Compound A. The compound of formula (I) is also known by the name "naporafenib". It will be understood that any reference to the term "Compound A" or "the compound of the formula (I)" herein includes the free form or a pharmaceutically acceptable salt thereof, unless otherwise indicated herein or otherwise clearly contradicted by context. The compound of formula (I) and its pharmaceutically acceptable salts are described in WO2014/151616, and methods of its preparation have been described, for example, in Example 1156 therein. In cell-based assays, the compound of formula (I) has demonstrated anti-proliferative activity in cell lines that contain a variety of mutations that activate MAPK signaling. In vivo, treatment with Compound A generated tumor regression in several KRAS-mutant models. Collectively, the *in vitro* and *in vivo* MAPK-pathway suppression and anti-proliferative activity observed for Compound A at well-tolerated doses suggests that Compound A may have anti-tumor activity in patients with tumors harboring activating lesions in the MAPK pathway. Moreover, Compound A is a Type 2 ATP-competitive inhibitor of both B-Raf and C-Raf that keeps the kinase pocket in an inactive conformation, thereby blocking mutant RAS-driven signaling and cell proliferation. Compound A has exhibited efficacy in numerous MAPK-driven human cancer cell lines and in xenograft tumors representing model tumors harboring human lesions in KRAS, NRAS and BRAF oncogenes.

The term "MEK inhibitor" is defined herein to refer to a compound which targets, decreases or inhibits at least one activity of MAP/ERK kinases 1 and 2 (MEK1/2). MEK stands for "mitogen-activated protein kinase".

The MEK inhibitor for use in the combination of the present invention is trametinib which has the formula (II): 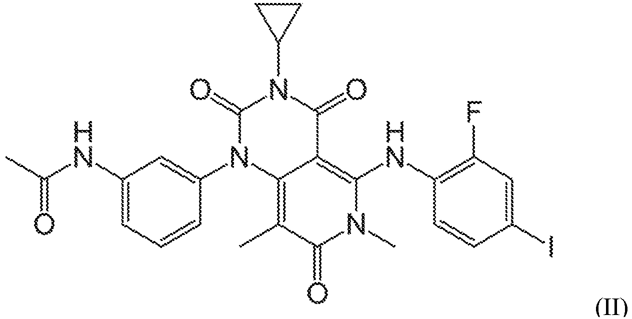 or is a pharmaceutically acceptable salt thereof, and/or is characterized by its name which is (N-(3-{3-cyclopropyl-5-[(2-fluoro-4-iodophenyl)amino]-6,8-dimethyl-2,4,7-trioxo-3,4,6,7-tetrahydropyrido[4,3-d]pyrimidin-1(2H)-yl } phenyl)acetamide), also referred to as JPT-74057 or GSK1120212, or a pharmaceutically acceptable salt or solvate thereof. The compound trametinib is disclosed in Example 4-1 in PCT Publication No. WO 2005/121142.

As a monotherapy trametinib has been approved for the treatment of unresectable or metastatic malignant melanoma with B-Raf V600E or V600K mutations and the compound is commercially available from Novartis AG under the trade name Mekinist^{®}.

Wherever "trametinib" is made reference to in the present invention disclosure, this refers to the free form or a pharmaceutically acceptable salt and/or a solvate of (N-(3- {3-cyclopropyl-5-[(2-fluoro-4-iodophenyl)amino]-6,8-dimethyl-2,4,7-trioxo-3,4,6,7-tetrahydropyrido[4,3-d]pyrimidin-1(2H)-yl}phenyl)acetamide).

The MEK inhibitor is trametinib, or a pharmaceutically acceptable salt or solvate thereof. In some embodiments, trametinib is in the form of a sodium salt. Suitably, trametinib is in the form of a solvate selected from: hydrate, acetic acid, ethanol, nitromethane, chlorobenzene, 1-pentancol, isopropyl alcohol, ethylene glycol and 3-methyl-1-butanol. In some preferred embodiments, trametinib is in the form of a dimethyl sulfoxide solvate. These solvates and salt forms can be prepared by one of skill in the art from the description in WO 2005/121142.

It will be understood that any reference to the term "trametinib" or "the compound of the formula (I)" herein includes the free form or a pharmaceutically acceptable salt or solvate, (particularly the dimethyl sulfoxide solvate) thereof, unless otherwise indicated herein or otherwise clearly contradicted by context.

As used herein the term "combination of the invention" refers to the combined administration comprised of (or especially consisting of) (a) a Raf inhibitor which is a Compound of formula (I), as defined herein, or a pharmaceutically acceptable salt thereof, and (b) the MEK inhibitor trametinib, or a pharmaceutically acceptable salt or solvate thereof. The Raf inhibitor Compound of formula (I), or a pharmaceutically acceptable salt thereof, and the MEK inhibitor trametinib, or a pharmaceutically acceptable salt or solvate thereof, may be employed in combination in accordance with the invention by administration simultaneously in a unitary pharmaceutical composition including both compounds. Alternatively, the combination may be administered separately in separate pharmaceutical compositions, each including Raf inhibitor and the MEK inhibitor in a sequential manner wherein, for example, the Raf inhibitor or the MEK inhibitor is administered first and the other second. Such sequential administration may be close in time (e.g., simultaneously) or remote in time.

As used herein, the terms "a" and "an" and "the" and similar references in the context of describing the invention are to be construed to cover both the singular and the plural, unless otherwise indicated herein or clearly contradicted by context. Where the plural form is used for compounds, salts, and the like, this is taken to mean also a single compound, salt, or the like.

The term "or" is used herein to mean, and is used interchangeably with, the term "and/or", unless context clearly indicates otherwise, e.g. reference to a compound or a pharmaceutically acceptable salt thereof relates to the free compound, a pharmaceutically acceptable salt, or a mixture of free compound and one or further more salts thereof.

"About" and "approximately" shall generally mean an acceptable degree of error for the quantity measured given the nature or precision of the measurements. Exemplary degrees of error are within 20 percent (%), typically, within 10%, and more typically, within 5% of a given value or range of values. When describing a dosage herein as "about" a specified amount, the actual dosage can vary by up to 10% from the stated amount: this usage of "about" recognizes that the precise amount in a given dosage form may differ slightly from an intended amount for various reasons without materially affecting the *in vivo* effect of the administered compound.

It will be understood that when a numerical value is given, without the term "about" preceding it, there will be a degree of variation associated with that given value, as is commonly accepted in the art.

As is customary in the art, dosages refer to the amount of the therapeutic agent in its free form. For example, when a dosage of about 0.5 mg of trametinib is referred to, and trametinib is used as its dimethyl sulfoxide solvate, the amount of the therapeutic agent used is equivalent to about 0.5 mg of the free form of trametinib.

The terms "comprising" and "including" or "with" are used herein in their open-ended and non-limiting sense unless otherwise noted, while "consisting of" limits the compound or components to those specifically mentiond. A pharmaceutical combination consisting of the Compound of formula (I), as defined herein (also named Compound A hereinafter), or a pharmaceutically acceptable salt thereof, and (b) trametinib, or a pharmaceutically acceptable salt or solvate thereof refers to a combination therapy where these two components are the only pharmaceutically active ingredients; with or without one or more pharmaceutically acceptable carrier materials which are administered for the treatment of a sarcoma, or rhabdomyosarcoma (in particular ERMS).

By "a combination" or "a pharmaceutical combination" (these terms being synonymous if not suggested otherwise) or "in combination with" or "combination therapy" it is not intended to imply that the therapy or the therapeutic agents must be physically mixed or administered at the same time and/or formulated for delivery together, although these methods of delivery are within the scope described herein. A therapeutic agent (especially Compound A and/or trametinib) in these combinations can be administered concurrently with, prior to, or subsequent to, one or more other additional therapies or therapeutic agents, or especially withouit such additional therapies or therapeutic agents (active ingredients). The therapeutic agents can be administered in any order. **In** general, each agent will be administered at a dose and/or on a time schedule determined for that agent. It will further be appreciated that the additional therapeutic agent utilized in this combination may be administered together in a single composition or administered separately in different compositions, especially where these time intervals allow that the combination partners show a cooperative, e.g., synergistic, effect, especially based on Bliss data. **In** general, it is expected that additional therapeutic agents utilized in combination be utilized at levels that do not exceed the levels at which they are utilized individually. **In** some embodyments, the levels utilized in combination will be lower than those utilized as single-agent therapeutics.

The term "synergistic effect" as used herein, refers to the action of Compound A and trametinib to produce an effect, for example, slowing the symptomatic progression of cancer or symptoms thereof, which is greater than the simple addition of the effects of each drug administered by themselves.

The term "combination therapy" preferably refers to the administration of two or more therapeutic agents to treat a therapeutic condition or disorder described in the present invention. Such administration encompasses co-administration of these therapeutic agents in a substantially simultaneous manner, such as in a single formulation having a fixed ratio of active ingredients or in separate formulations (e.g., capsules and/or intravenous formulations) for each active ingredient. **In** addition, such administration also encompasses use of each type of therapeutic agent in a sequential or separate manner, either at approximately the same time or at different times. Regardless of whether the active ingredients are administered as a single formulation or in separate formulations, the drugs are administered to the same patient as part of the same course of therapy. **In** any case, the treatment regimen will provide beneficial effects in treating the conditions or disorders described herein.

By simultaneous therapeutic use, within the meaning of the present invention is meant in particular an administration of at least two active ingredients by the same route and at the same time or at substantially the same time.

By separate use, within the meaning of the present invention is meant in particular an administration of at least two active ingredients at the same time or at substantially the same time by different routes.

By sequential therapeutic use is meant in particular administration of at least two active ingredients at different times, the administration route being identical or different. More particularly by an administration method is meant according to which the whole administration of one of the active ingredients is carried out before administration of the other or others commences.

The terms "fixed combination", "fixed dose" and "single formulation" as used herein preferably refers to one dosage form formulated to deliver an amount, which is jointly therapeutically effective for the treatment of cancer, of both therapeutic agents to a patient. The single vehicle is designed to deliver an amount of each of the agents along with any pharmaceutically acceptable carriers (excipients). **In** some embodiments, the vehicle is a tablet, capsule, pill, or a patch. **In** other embodiments, the vehicle is a solution or a suspension.

The term combination also or alternatively includes a "non-fixed combination" or "kit of parts" means that the therapeutic agents of the combination of the invention are both administered to a patient as separate entities either simultaneously, concurrently or sequentially with no specific time limits, especially so that they are jointly active, wherein such administration provides therapeutically effective levels of the two compounds in the body of a patient in need thereof. The latter further also applies to cocktail therapy, e.g., the administration of three or more active ingredients.

The term "pharmaceutically acceptable" as used herein refers to those compounds, materials, compositions and/or dosage forms, which are, within the scope of sound medical judgment, suitable for contact with the tissues of a patient, *e.g.,* an adult patient or a human patient, without excessive toxicity, irritation, allergic response and other problems or complications commensurate with a reasonable benefit/risk ratio.

As used herein, the term "pharmaceutically acceptable excipient" or "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art. Except insofar as any conventional carrier is incompatible with the active ingredient, its use in the therapeutic or pharmaceutical compositions is contemplated.

The term "pharmaceutical composition" is defined herein to refer to a mixture or solution containing at least one therapeutic agent to be administered to a patient, *e.g.,* a mammal or human, in order or treat a particular disease or condition affecting the patient. The present pharmaceutical combinations can be formulated in suitable pharmaceutical compositions for enteral or parenteral administration, such as sugar-coated tablets, tablets, capsules or suppositories, or ampoules. If not indicated otherwise, these are prepared in a manner known per se, for example by means of various conventional mixing, comminution, direct compression, granulating, sugar-coating, dissolving, lyophilizing processes, or fabrication techniques readily apparent to those skilled in the art. It will be appreciated that the unit content of a combination partner contained in an individual dose of each dosage form need not in itself constitute an effective amount since the necessary effective amount may be reached by administration of a plurality of dosage units. The pharmaceutical composition may contain, from about 0.1 % to about 99.9 %, preferably from about 1 % to about 60 %, of each of the therapeutic agents, summing up to an amount of 0.2 % to 70 %. One of ordinary skill in the art may select one or more of the aforementioned carriers with respect to the particular desired properties of the dosage form by routine experimentation and without any undue burden. The amount of each carriers used may vary within ranges conventional in the art. The following references disclose techniques and excipients used to formulate oral dosage forms: The Handbook of Pharmaceutical Excipients, 4th edition, Rowe et al., Eds., American Pharmaceuticals Association (2003); and Remington: the Science and Practice of Pharmacy, 20th edition, Gennaro, Ed., Lippincott Williams & Wilkins (2003). These optional additional conventional carriers may be incorporated into the oral dosage form either by incorporating the one or more conventional carriers into the initial mixture before or during granulation or by combining one or more conventional carriers with granules comprising the combination of agents or individual agents of the combination of agents in the oral dosage form. **In** the latter embodiment, the combined mixture may be further blended, e.g., through a V-blender, and subsequently compressed or molded into a tablet, for example a monolithic tablet, encapsulated by a capsule, or filled into a sachet.

Where "%" is mentioned, this refers to weight percent if not indicated otherwise.

Pharmaceutical compositions may be presented in unit dose forms containing a predetermined amount of active ingredient per unit dose. **In** certain embodiments, the unit dose includes one or more vehicles such that each vehicle includes an effective amount of at least one of the therapeutic agents along with one or more pharmaceutically acceptable carriers and excipients. **In** some embodiments, the unit dose is one or more tablets, capsules, pills, injections, infusions, patches, or the like, administered to the patient at the same time. As is known to those skilled in the art, the amount of active ingredient per dose will depend on the condition being treated, the route of administration and the age, weight and condition of the patient. Preferred unit dosage compositions are those containing a daily dose or sub-dose, or an appropriate fraction thereof, of an active ingredient. Furthermore, such pharmaceutical compositions may be prepared by any of the methods well known in the pharmacy art.

The pharmaceutical combinations and compositions of the invention may include a "therapeutically effective amount" or "effective amount" of a compound of the invention. The term "pharmaceutically effective amount", "therapeutically effective amount" or "clinically effective amount" of a combination of therapeutic agents is an amount sufficient, at dosages and for periods of time necessary, to provide an observable or clinically significant improvement over the baseline of clinically observable signs and symptoms of the disorders treated with the combination. A therapeutically effective amount may vary according to factors such as the disease state, age, sex, and weight of the individual. A therapeutically effective amount is also one in which any toxic or detrimental effects of the therapeutic agents are outweighed by therapeutically beneficial effects. A "therapeutically effective dosage" preferably modulates a measurable parameter, such as tumor growth rate or disease progression in a desired manner. The ability of a compound to modulate a measurable parameter can be evaluated in an animal model system predictive of efficacy in human sarcomas to help establish suitable dosing levels and schedules. Alternatively, this property of a composition can be evaluated by examining the ability of the compound to modulate an undesired parameter by using in vitro assays known to the skilled practitioner.

Preferably, the following dosages are used in combinations for Compound A and trametinib according to the present invention in the case of treatment of human patients.

A unit dosage of the active ingredients may be administered once daily, or twice daily, or three times daily, or four times daily, in sum yielding the appropriate daily dosage, e.g. as inmdicated below, with the actual dosage and timing of administration determined by criteria such as the patient's age, weight, and gender; the extent and severity of the cancer to be treated; and the judgment of a treating physician. Oral dosage forms are preferred.

Where doses or dosages are mentioned herein, the amount referred to refers to the amount of the therapeutic agent (active ingredient, here in free form). For example, when a 2 mg dosage of trametinib is administered, and trametinib is administered in a tablet containing trametinib dimethyl sulfoxide, the tablet will contain trametinib dimethyl sulfoxide equivalent to 2 mg trametinib.

Compound A is preferably administered orally. In a preferred embodiment, the Raf inhibitor, especially Compound A, or a pharmaceutically acceptable salt thereof, respectively, is administered at a total daily dose (TTD) of from about 200 mg to about 1200 mg. The total daily dose of Compound A is administered in one or more dosage units daily. Preferably the total daily dose of Compound A is administered twice daily. Thus, Compound A may be administered from about 100 mg to about 600 mg twice daily.

In some embodiments, Compound A is administered (preferably orally) once daily (QD) at a dose of about, respectively, 100 mg, 125 mg, 150 mg, 175 mg, 200 mg, 225 mg, 250 mg, 275 mg, 300 mg, 325 mg, 350 mg, 375 mg or 400 mg.

In some embodiments, Compound A is administered (preferably orally), at a dosage of about 100 mg, about 200 mg, about 300 mg, about 400 mg or about 600 mg twice daily (BID or bid).

The total daily dose of trametinib is preferably administered once daily (QD or qd) and is less than about 2 mg per day.

In a preferred embodiment, the MEK inhibitor trametinib, or a pharmaceutically acceptable salt or solvate (e.g. the dimethyl sulfoxide solvate) thereof, is administered (preferably orally) as part of the combination according to the present invention (especially to a patient in need thereof) in a total daily dose which is from about 0.25 mg to about 2 mg per day; suitably, the amount will be selected from about 0.5 mg to about 2 mg per day. Suitably, the amount will be about 0.5 mg, about 1 mg, about 1.5 mg or about 2 mg per day. In a preferred embodiment, trametinib, or a pharmaceutically acceptable salt or solvate thereof, is administered at total daily dose of 0.5 mg or 1.5 mg or 2 mg per day. The daily dosages may be distributed over one to two, preferably one dosage units.

The daily dose of Compound of formula (I) may be preferentially administered twice daily, whilst the daily dose of trametinib is preferntially administered once daily.

In one embodiment, Compound A may be administered in a TDD which is from about 400 to about 800 mg (e.g., from about 200 mg to about 400 mg, administered twice daily) and trametinib may be administered in a TDD of about 1 mg or less than about 1 mg, preferably once a day.

In one embodiment, Compound A may be administered in a TDD which is from about 400 to about 800 mg (e.g., from about 200 mg to about 400 mg, administered twice daily) and trametinib may be administered in a TDD of about 0.5 mg or less than about 0.5 mg, preferably once a day.

In one embodiment, Compound A may be administered in a TDD which is from about 400 to about 800 mg (e.g., from about 200 mg to about 400 mg, administered twice daily) and trametinib may be administered in a TDD of about 1.5 mg or less than about 1.5 mg, preferably once a day.

In addition, an efficacious dosage can be determined by monitoring biomarkers indicative of MAP kinase pathway inhibition. In particular, *DUSP6* is a known biomarker for this pathway.

A further benefit may be that lower doses of the therapeutic agents of the combination of the invention can be used, for example, such that the dosages may not only often be smaller, but also may be applied less frequently, or can be used in order to diminish the incidence of side-effects observed with one of the combination partners alone. This is in accordance with the desires and requirements of the patients to be treated.

The term "jointly therapeutically active" or "joint therapeutic effect" as used herein means that the therapeutic agents can be given jointly, separately or sequentially in such time intervals such that the patient, especially human, to be treated, still shows an (preferably synergistic) interaction (joint therapeutic effect). Whether this is the case can, *inter alia,* be determined by following the blood levels of the compounds, showing that both compounds are present in the blood of the human to be treated at least during certain time intervals, or by the fact that subsequent treatment leads to a better result than single treatment with only one of the active ingredients.

The term "active ingredient" or "therapeutic agent" comprises the Raf inhibitor and the Mek inhibitor as defined for combinations according to the invention embodiments.

As used herein the term "agent" is understood to mean a substance that produces a desired effect in a tissue, system, animal, mammal, human, or other patient. It is also to be understood that an "agent" may be a single compound or a combination or composition of two or more compounds.

As used herein, the term "sarcoma" refers to a disease characterized by the undesired and uncontrolled growth of aberrant cells. Sarcoma cells can spread locally or through the bloodstream and lymphatic system to other parts of the body. As used herein, the term "sarcoma" includes premalignant, as well as malignant sarcomas. Preferably, the term relates to RAS mutant (especially NRAS- or HRAS-mutant) sarcoma, more preferably to soft tissue sarcoma or yet more preferably to undifferentiated pleomorphic sarcoma (UPS). Most preferably, the term "sarcoma" refers to rhabdomyosarcoma, especially to ERMS, where most especially ERMS refers to RAS-mutant ERMS.

An "oral dosage form" includes a unit dosage form prescribed or intended for oral administration.

As used herein, the terms "therapy", "treat", "treatment" and "treating" refer to the reduction or amelioration of the progression, severity and/or duration of sarcoma, or the amelioration of one or more symptoms, suitably of one or more discernible symptoms, of the disorder resulting from the administration of one or more therapies. In specific embodiments, the terms "therapy", "treat", "treatment" and "treating" refer to the amelioration of at least one measurable physical parameter of sarcoma, such as growth of a sarcoma tumor, not necessarily discernible by the patient. In other embodiments the terms "therapy", "treat", "treatment" and "treating" refer to the inhibition of the progression of sarcoma, either physically by, *e.g.,* stabilization of a discernible symptom, physiologically by, *e.g.,* stabilization of a physical parameter, or both. In other embodiments the terms "therapy", "treat", "treatment" and "treating" refer to the reduction or stabilization of tumor size or cancerous cell count in sarcoma.

Within the meaning of the present disclosure, the term "treat" also denotes to arrest, delay the onset (i.e., the period prior to clinical manifestation of a disease) and/or reduce the risk of developing or worsening a disease. The term "protect" is used herein to mean prevent, delay, or treat, or all, as appropriate, development, continuance or aggravation of a disease in a patient..

The term "patient" or "patient" or "human patient" as used herein is intended to include a patient who is capable of suffering from or afflicted with a cancer or any disorder involving, directly or indirectly, a sarcoma.

The combination of the invention is particularly useful in the treatment of young adults and pediatric patients (including adolescent patients). A pediatric patient is a person with an age of 18 years or less. A pediatric patient is also a child < 1 year of age, or 1-9 years, or a patient between the ages of 10 to 18.

The patient to be treated by the combination of the invention may also be less than 15 years or between the age of 15-29 years.

The term "inhibition", "inhibitor," or "antagonist" includes a reduction in a certain parameter, e.g., an activity, of a given molecule or pathway. For example, inhibition of an activity of a targeted kinase (Raf or MEK) by 5%, 10%, 20%, 30%, 40% or more is included by this term. Thus, inhibition may be, but need not be, 100%.

As used herein, "salts" (which, what is meant by "or salts thereof" or "or a salt thereof"), can be present alone or in mixture with free compounds of the combination of the invention, e.g., Raf inhibitor Compound with formula (I) or trametinib, and are preferably pharmaceutically acceptable salts. Such salts are formed, for example, as acid addition salts, preferably with organic or inorganic acids, from compounds of the combination of the invention with a basic nitrogen atom, especially the pharmaceutically acceptable salts. The term "pharmaceutically acceptable salts" refers to salts that retain the biological effectiveness and properties of the compound and which typically are not biologically or otherwise undesirable. The compound may be capable of forming acid addition salts by virtue of the presence of an amino group.

Lists of suitable salts can be found, *e.g.,* in "Remington's Pharmaceutical Sciences", Mack Publishing Company, Easton, Pa.; and in "Handbook of Pharmaceutical Salts: Properties, Selection, and Use" by Stahl and Wermuth (Wiley-VCH, Weinheim, Germany, 2008).

For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred. **In** view of the close relationship between the novel compounds in free form and those in the form of their salts, including those salts that can be used as intermediates, for example in the purification or identification of the novel compounds, any reference to the free compounds is to be understood as referring also to the corresponding salts, as appropriate and expedient. The salts of compounds used in the combination of the invention are preferably pharmaceutically acceptable salts; suitable counter-ions forming pharmaceutically acceptable salts are known in the field. Unless otherwise specified, or clearly indicated by the text, reference to therapeutic agents useful in the pharmaceutical combination provided herein includes both the free base of the compounds, and all pharmaceutically acceptable salts of the compounds. As used herein, the term "solvate" refers to a complex of variable stoichiometry formed by a solute (in this invention, trametinib) or a salt thereof and a solvent. Such solvents for the purpose of the invention may not interfere with the biological activity of the solute. Examples of suitable solvents include, but are not limited to, water, methanol, dimethylsulforide, ethanol and acetic acid. Examples of suitable pharmaceutically acceptable solvents include, without limitation, water, ethanol and acetic acid.

The term "synergistic effect" as used herein, refers to action of two agents, the Raf inhibitor Compound with formula (I), or a pharmaceutically acceptable salt thereof, and the MEK inhibitor trametinib, to produce an effect, for example, slowing the symptomatic progression of cancer or symptoms thereof, which is greater than the simple addition of the effects of each drug administered by themselves.

Sarcomas to be treated by the pharmaceutical combination of the invention are in particular HRAS-mutant sarcomas. The term "HRAS-mutant" sarcoma includes any sarcoma that exhibits a mutated HRAS protein, in particular gain of function HRAS-mutation; especially any of G13R, Q61K, or further Q61K, Q61H orQ61R, HRAS-mutant sarcoma. The sarcoma may e.g. be locally advanced or metastatic.

Sarcomas to be treated by the pharmaceutical combination of the invention are in particular NRAS-mutant sarcomas. The term "NRAS-mutant" sarcoma includes any sarcoma that exhibits a mutated NRAS protein, e.g., Q61K. The sarcoma may e.g. be locally advanced or metastatic.

In another invention embodiment, the sarcoma, especially rhabdomyosarcoma, most especially ERMS, to be treated according to the invention is resistant or refractory to standard of care, e.g. surgery or alkylation-based multidrug chemotherapy. Such chemotherapy may comprise (i) one or more of ifosfamide, vincristine and dactinomycin, or combinations thereof, (ii) one or more of vincristine, dactinomycin and cyclophosphamide, or combinations thereof, or (iii) a regimen comprising irinotecan-vincristine plus a combination of vincristine, dactinomycin and cyclophosphamide.

### BRIEF DESCRIPTION OF FIGURES

Fig. 1 represents the treatment of a tumor xenograft of a HRAS G13R mutant rhabdomyosarcoma in scid mice and the body weight change in mice following treatment - for details see the Examples section below.
Fig. 2 represents the treatment of a tumor xenograft of a NRAS Q61K mutant rhabdomyosarcoma in scid mice and the body weight change in mice following treatment - for details see the Examples section below.
Fig. 3 represents the treatment of a tumor xenograft of a HRAS Q61K mutant rhabdomyosarcoma in scid mice - for details see the Examples section below.
Fig. 4 represents the treatment of a tumor xenograft of a HRAS Q61K mutant rhabdomyosarcoma in scid mice - for details see the Examples section below.
Fig. 5 represents the % body weight change in tumor xenograft of HRAS G13R mutant rhabdomyosarcoma in scid mice. Compound A is dosed at 50 mg/kg bid when trametinib is dosed at 0.0375 mg/kg qd. Compound A is dosed at 25 mg/kg bid when trametinib is dosed at 0.075mg/kg qd.
Fig. 6 represents the treatment of a tumor xenograft of a HRAS G13R mutant rhabdomyosarcoma in scid mice - for details see the Examples section below.
Fig. 7 represents the % body weight change in tumor xenograft of HRAS G13R mutant rhabdomyosarcoma in scid mice. Compound A is dosed at 50 mg/kg bid when trametinib is dosed at 0.0375 mg/kg qd. Compound A is dosed at 25 mg/kg bid when trametinib is dosed at 0.075mg/kg qd.
Fig. 8 represents the treatment of a tumor xenograft of a HRAS Q61K mutant rhabdomyosarcoma in scid mice with Compound A. Tumor treated with Compound A gradually acquires resistance, becoming almost completely resistant at round 3 of treatment.
Fig. 9 represents the treatment of a tumor xenograft of a HRAS Q61K mutant rhabdomyosarcoma in scid mice with Compound A in combination with trametinib and shows that even after three rounds no resistance emerges - for details see the Examples section below.

### EXAMPLES

The Examples below are set forth to aid in the understanding of the invention but are not intended, and should not be construed, to limit its scope in any way.

### Example 1: Antitumor efficacy of Compound A alone and in combination with trametinib

The *in vivo* antitumor efficacy of the B/CRAF inhibitor LX254 (Compound A) alone or in combination with trametinib was evaluated in three ERMS patient-derived xenograft models (PDX) and one cell line-derived xenograft model (CDX). All models harbor oncogenic H- or N-RAS mutations as follows:
- SJRHB013758 PDX (HRAS G13R),
- SJRHB013 PDX (NRAS Q61K),
- SJRHB000026 PDX (HRAS G13R),
- SMS **CTR** CDX (HRAS Q61K).

The three PDX models were acquired from the childhood solid tumor network (Stewart, E et al. "The childhood solid tumor network: A new resource for the developmental biology and oncology research communities." Dev. Biol. 2016."). SMS-CTR cell line were donated by Dr.Corinne Linardic, Duke University.

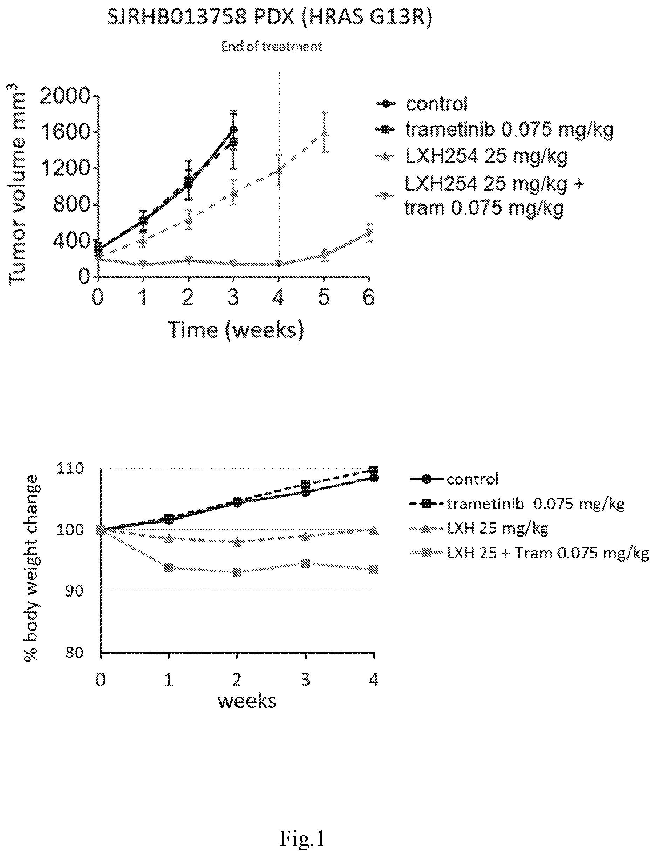

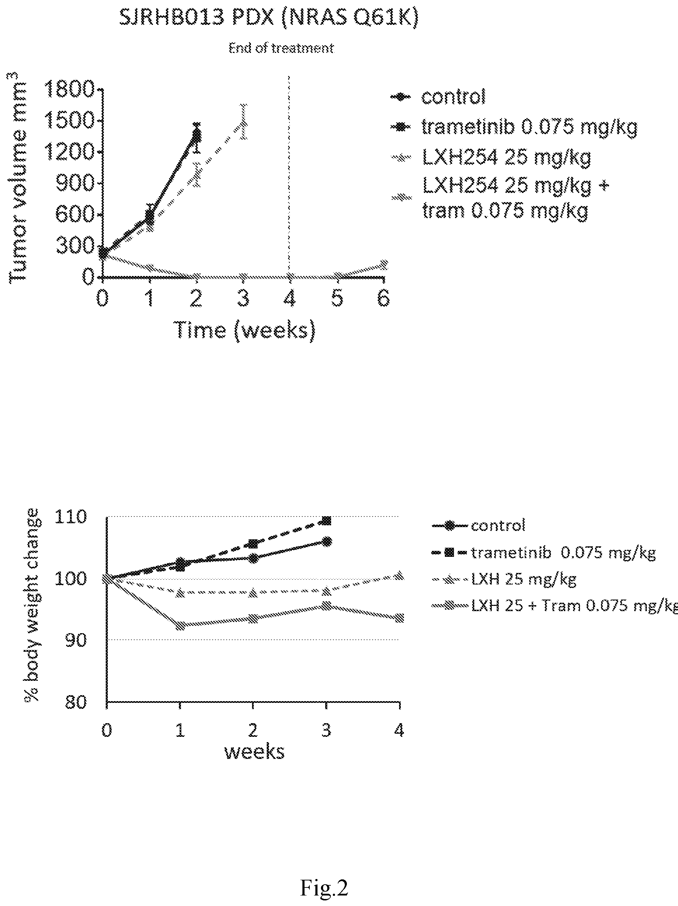

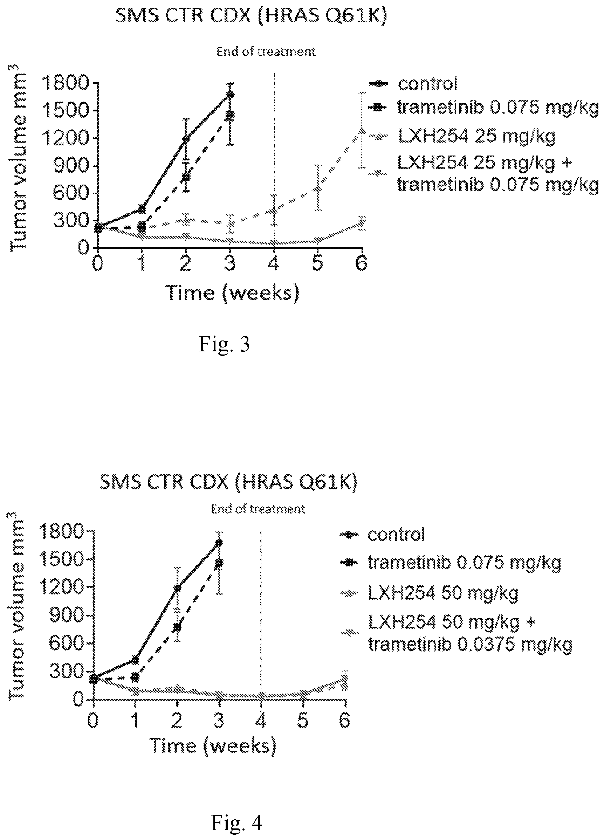

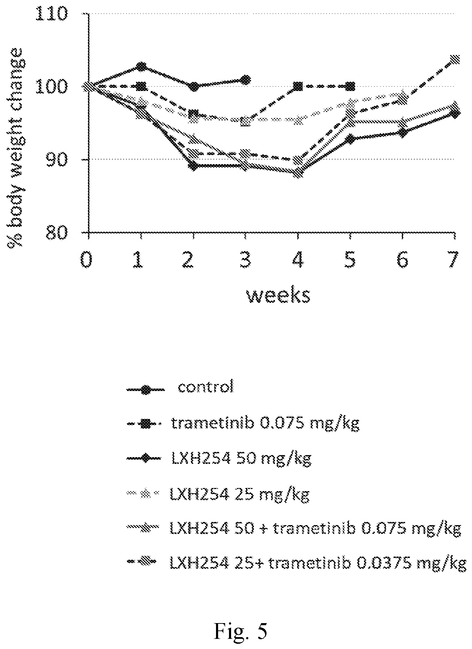

Small tumor tissue pieces from the indicated PDX/CDX models were implanted under the skin in the flank area of C.B.17-scid mice to generate treatment cohorts to test for *in vivo* antitumor efficacy. When tumors reached approximately 300 mm³, mice were treated with LXH254 (25 mg/kg or 50 mg/kg twice a day), trametinib (0.075 mg/kg once a day), or the combination of both at LXH254 (25 mg/kg or 50 mg/kg twice a day), trametinib (0.075 mg/kg or 0.0375 mg/kg once a day) (Figures 1-7). For SMS-CTR xenografts (figures 3 and 4), dosing *was per os,* with LXH254 as a solution in 0.5% DMSO and 20% Captisol and trametinib was administered as a solution in 20% methyl cellulose. For remaining xenografts (Figures 1, 2, 4-6) dosing was *per os,* with LXH254 in MEPC 4 (microemulsion preconcentrate) vehicle and trametinib in 0.2% Tween 80 + 0.5% HPMC in Water; adjusted to pH 8.

Duration of the treatment was 4 weeks and tumor volume was measured once a week with digital calipers. Mice were sacrificed when tumors reached 2 cm³ volume In control, trametinib, and sometimes LXH54 groups, tumors reached maximum allowed volume of 2cm³ and had to be sacrificed before the end of 4 weeks.

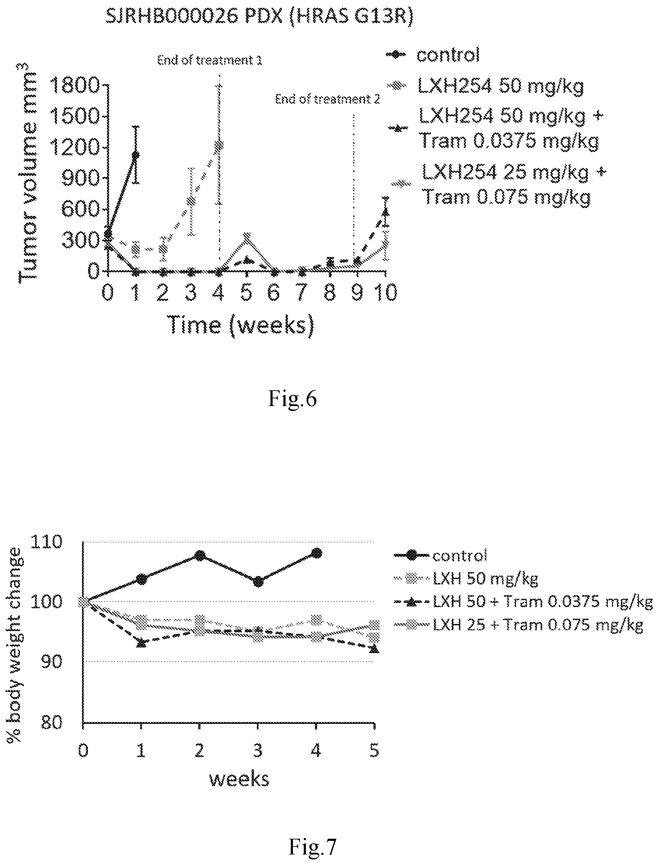

As shown in Figures 1-4, while single treatments had minimal effect on tumor growth, the combination of LXH254 with trametinib caused tumor regression in all models shown, with complete regression seen in SJRHB013 PDX and SJRHB000026 PDX models. In SMS-CTR xenografts, administration of LXH254 50 mg/kg bid caused regression to a smilar extent as compared to LXH254 50 mg/kg bid + trametinib 0.0375 mg/kg qd ( Figure 4). In SJRHB000026 PDX, a second round or treatment was initiated after tumors regrew to approximately 300mm³ post first round of treatment. Complete regression was seen again (Figure 6). In SJRHB000026, both combinatorial schedules (LXH254 25 mg/kg bid + trametinib 0.075 mg/kg qd and LXH254 50 mg/kg bid + trametinib 0.0375 mg/kg qd) caused complete regression, unlike administration of LXH254 50 mg/kg bid alone.

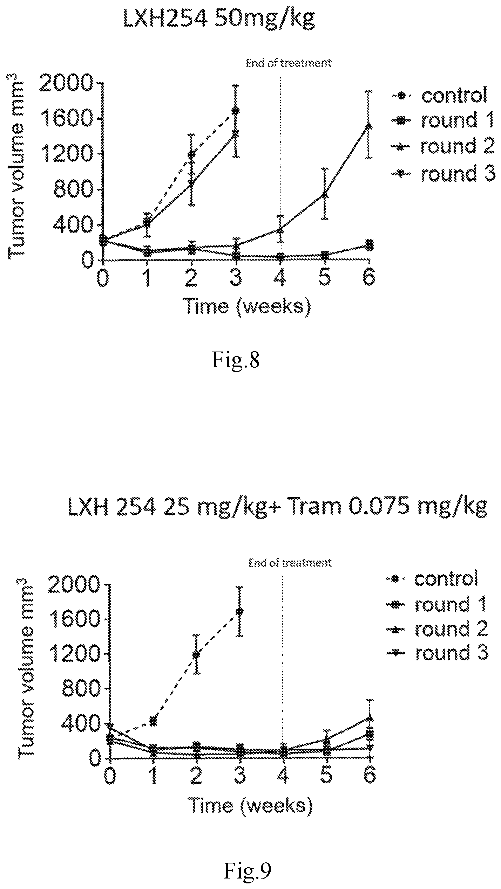

In addition, the rate of resistance acquisition to LXH254 alone or LXH254 in combination with trametinib was compared in the SMS-CTR CDX model. For assessing acquisition of treatment resistance (Figure 8 and Figure 9), SMS-CTR xenografts were generated and treated with 50 mg/kg LXH254 bid or combination LXH254 25mg/kg bid + trametinib 0.075 mg/kg qd as done with the models in figures 1-4. After 4 weeks of treatment (round 1), tumors were allowed to regrow post treatment and re-transplanted in new cohort of mice. At 300 mm³ tumor size, a second round of treatment was initiated with the same doses (round 2). Round 3 was initiated as above from round 2 post-treatment tumors. Tumors treated with 50mg/kg LXH254 bid (Figure 8) gradually acquired resistance, becoming almost completely resistant at round 3 of treatment (see top 2 curves of Figure 8, where the response to LXH254 is similar to the treatment with control). In contrast, as shown in Figure 9, tumors treated with the combination LXH254 (25mg/kg bid) + trametinib (0.075 mg/kg qd) did not acquire resistance up to round 3 and continued to show tumor regression for the first 4-5 weeks.

### Example 2: Tolerability of treatment of a combination of Compound A and trametinib in RAS-mutant ERMS xenografts

The weight of mice treated as described in Example 1 were measuerd daily to ensure accurate dosing and to monitor the treatment for toxicity.

Mice treated with the combinaton of LXH254 with trametinib, dosed at either 25 mg/kg bid (LXH254) +0.075mg/kg qd (trametinib) or 50 mg/kg bid (LXH254) + 0.0375 mg/kg qd (trametinib) exhibited between 5-10% body weight loss (Figures 1, 2, 5 and 7). However the combination was overall well tolerated.

## Claims

1. A pharmaceutical combination comprising:
(a) a Raf inhibitor which is a Compound of formula (I), 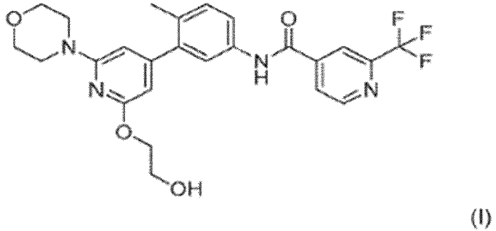 or a pharmaceutically acceptable salt thereof, and
(b) trametinib, or a pharmaceutically acceptable salt or solvate thereof, for use in the treatment of sarcoma, especially RAS-mutant sarcoma.

2. The pharmaceutical combination for use according to claim 1, wherein the sarcoma is rhabdomyosarcoma.

3. The pharmaceutical combination for use according to claim 1, wherein the sarcoma is embryonal rhabdomyosarcoma (ERMS).

4. The pharmaceutical combination for use according to claim 1, 2 or 3, wherein a patient suffering from the sarcoma is a pediatric patient or a young adult patient.

5. The pharmaceutical combination for use according to any one of claims 1 to 4, wherein the compound of formula (I), or a pharmaceutically acceptable salt thereof, is administered at a total daily dose of about from about 200 mg to about 1200 mg.

6. The pharmaceutical combination for use according to any one of claims 1 to 5, wherein the total daily dose of the compound of formula (I) is administered twice daily, in two equal doses.

7. The pharmaceutical combination for use according to any one of claims 1 to 6, wherein the compound of formula (I) is administered in an amount of from about 200 to 400 mg twice daily.

8. The pharmaceutical combination for use according to any one of claims 1 to 7, wherein trametinib, or a pharmaceutically acceptable salt, or a solvate thereof, is administered in an amount of from about 0.5 to about 2 mg per day, preferably once daily.

9. The pharmaceutical combination for use according to any one of claims 1 to 7, wherein trametinib, or a pharmaceutically acceptable salt, or a solvate thereof, is administered in an amount of from about 0.5, about 1.0 or about 1.5 mg per day, preferably once daily.

10. The pharmaceutical combination for use according to any one of claims 1 to 8, wherein the Compound of formula (I), or a pharmaceutically acceptable salt thereof, and trametinib, or a pharmaceutically acceptable salt thereof, or a solvate thereof, are administered so that they are jointly active, especially in a synergistic manner.

11. The pharmaceutical combination for use according to any one of claims 1 to 10 in the form of a kit of parts, comprising of unit dosage forms with the compound of the formula (I), or a pharmaceutically acceptable salt thereof, and trametinib, or a pharmaceutically acceptable salt thereof, or a solvate thereof, in separate formulations and/or in combined formulations, for simultaneous, concurrent or sequential administration, especially so that they are jointly effective; said kit optionally also including instructions for use.

12. A Raf inhibitor which is a Compound of formula (I) as defined in claim 1, or a pharmaceutically acceptable salt thereof, for use in a combination therapy with trametinib, or a pharmaceutically acceptable salt or solvate thereof, in the treatment of sarcoma (such as RAS-mutant sarcoma), especially rhabdomyosarcoma or more especially embryonal rhabdomyosarcoma (ERMS).

13. Trametinib, or a pharmaceutically acceptable salt or solvate thereof, for use in a combination therapy with a Raf inhibitor which is a Compound of formula (I) as defined in claim 1, or a pharmaceutically acceptable salt thereof, in the treatment of sarcoma (such as RAS-mutant sarcoma), especially rhabdomyosarcoma or more especially embryonal rhabdomyosarcoma (ERMS).

## Patentansprüche

1. Pharmazeutische Kombination, umfassend:
(a) einen Raf-Inhibitor, der eine Verbindung der Formel (I) ist, 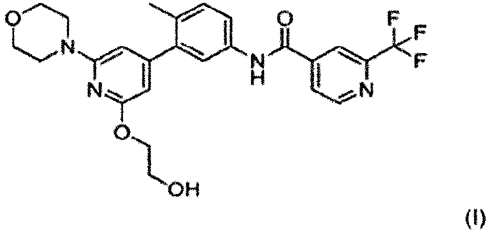 oder ein pharmazeutisch unbedenkliches Salz davon, und
(b) Trametinib oder ein pharmazeutisch unbedenkliches Salz oder Solvat davon zur Verwendung bei der Behandlung von Sarkom, insbesondere RAS-mutiertem Sarkom.

2. Pharmazeutische Kombination zur Verwendung nach Anspruch 1, wobei das Sarkom Rhabdomyosarkom ist.

3. Pharmazeutische Kombination zur Verwendung nach Anspruch 1, wobei das Sarkom embryonales Rhabdomyosarkom (ERMS) ist.

4. Pharmazeutische Kombination zur Verwendung nach Anspruch 1, 2 oder 3, wobei ein Patient, der an dem Sarkom leidet, ein pädiatrischer Patient oder ein junger erwachsener Patient ist.

5. Pharmazeutische Kombination zur Verwendung nach einem der Ansprüche 1 bis 4, wobei die Verbindung der Formel (I), oder ein pharmazeutisch unbedenkliches Salz davon, in einer täglichen Gesamtdosis von etwa 200 mg bis etwa 1200 mg verabreicht wird.

6. Pharmazeutische Kombination zur Verwendung nach einem der Ansprüche 1 bis 5, wobei die tägliche Gesamtdosis der Verbindung der Formel (I) zweimal täglich in zwei gleichen Dosen verabreicht wird.

7. Pharmazeutische Kombination zur Verwendung nach einem der Ansprüche 1 bis 6, wobei die Verbindung der Formel (I) in einer Menge von etwa 200 bis 400 mg zweimal täglich verabreicht wird.

8. Pharmazeutische Kombination zur Verwendung nach einem der Ansprüche 1 bis 7, wobei Trametinib oder ein pharmazeutisch unbedenkliches Salz oder ein Solvat davon in einer Menge von etwa 0,5 bis etwa 2 mg pro Tag, vorzugsweise einmal täglich, verabreicht wird.

9. Pharmazeutische Kombination zur Verwendung nach einem der Ansprüche 1 bis 7, wobei Trametinib oder ein pharmazeutisch unbedenkliches Salz oder ein Solvat davon in einer Menge von etwa 0,5, etwa 1,0 oder etwa 1,5 mg pro Tag, vorzugsweise einmal täglich, verabreicht wird.

10. Pharmazeutische Kombination zur Verwendung nach einem der Ansprüche 1 bis 8, wobei die Verbindung der Formel (I), oder ein pharmazeutisch unbedenkliches Salz davon, und Trametinib oder ein pharmazeutisch unbedenkliches Salz davon oder ein Solvat davon so verabreicht werden, dass sie gemeinsam aktiv sind, insbesondere auf synergistische Weise.

11. Pharmazeutische Kombination zur Verwendung nach einem der Ansprüche 1 bis 10 in Form eines Kits von Teilen, umfassend Einheitsdosierungsformen mit der Verbindung der Formel (I) oder einem pharmazeutisch unbedenklichen Salz davon und Trametinib oder einem pharmazeutisch unbedenklichen Salz davon oder einem Solvat davon in separaten Formulierungen und/oder in kombinierten Formulierungen zur simultanen, zeitgleichen oder sequentiellen Verabreichung, so dass sie insbesondere gemeinsam wirksam sind; wobei das Kit gegebenenfalls auch Gebrauchsanweisungen einschließt.

12. Raf-Inhibitor, der eine Verbindung der Formel (I) wie in Anspruch 1 definiert ist, oder ein pharmazeutisch unbedenkliches Salz davon, zur Verwendung in einer Kombinationstherapie mit Trametinib oder einem pharmazeutisch unbedenklichen Salz oder Solvat davon bei der Behandlung von Sarkom (wie RAS-mutiertem Sarkom), insbesondere Rhabdomyosarkom oder spezieller embryonalem Rhabdomyosarkom (ERMS).

13. Trametinib oder ein pharmazeutisch unbedenkliches Salz oder Solvat davon zur Verwendung in einer Kombinationstherapie mit einem Raf-Inhibitor, der eine Verbindung der Formel (I) wie in Anspruch 1 definiert ist, oder einem pharmazeutisch unbedenklichen Salz davon, bei der Behandlung von Sarkom (wie RAS-mutiertem Sarkom), insbesondere Rhabdomyosarkom oder spezieller embryonalem Rhabdomyosarkom (ERMS).

## Revendications

1. Combinaison pharmaceutique comprenant :
(a) un inhibiteur de Raf qui est un composé de formule (I), 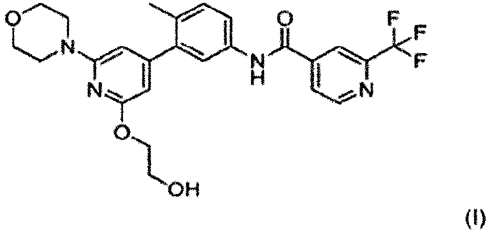 ou un sel pharmaceutiquement acceptable de celui-ci, et
(b) du tramétinib, ou un sel ou solvate pharmaceutiquement acceptable de celui-ci, pour une utilisation dans le traitement d'un sarcome, en particulier d'un sarcome porteur d'une mutation de RAS.

2. Combinaison pharmaceutique selon la revendication 1, le sarcome étant un rhabdomyosarcome.

3. Combinaison pharmaceutique selon la revendication 1, le sarcome étant un rhabdomyosarcome embryonnaire (ERMS).

4. Combinaison pharmaceutique selon la revendication 1, 2 ou 3, un patient souffrant du sarcome étant un enfant ou un jeune adulte.

5. Combinaison pharmaceutique selon l'une quelconque des revendications 1 à 4, le composé de formule (I) ou un sel pharmaceutiquement acceptable de celui-ci, étant administré à une dose journalière totale d'environ 200 mg à environ 1 200 mg.

6. Combinaison pharmaceutique selon l'une quelconque des revendications 1 à 5, la dose journalière totale du composé de formule (I) étant administrée deux fois par jour, en deux doses égales.

7. Combinaison pharmaceutique selon l'une quelconque des revendications 1 à 6, le composé de formule (I) étant administré en une quantité d'environ 200 à 400 mg deux fois par jour.

8. Combinaison pharmaceutique selon l'une quelconque des revendications 1 à 7, le tramétinib, ou un sel pharmaceutiquement acceptable ou un solvate de celui-ci, étant administré en une quantité d'environ 0,5 à environ 2 mg par jour, de préférence une fois par jour.

9. Combinaison pharmaceutique selon l'une quelconque des revendications 1 à 7, le tramétinib, ou un sel pharmaceutiquement acceptable ou un solvate de celui-ci, étant administré en une quantité d'environ 0,5, environ 1,0 ou environ 1,5 mg par jour, de préférence une fois par jour.

10. Combinaison pharmaceutique selon l'une quelconque des revendications 1 à 8, le composé de formule (I) est : ou un sel pharmaceutiquement acceptable de celui-ci , et le tramétinib, ou un sel pharmaceutiquement acceptable de celui-ci ou un solvate de celui-ci, étant administrés de sorte qu'ils soient conjointement actifs, notamment de façon synergique.

11. Combinaison pharmaceutique selon l'une quelconque des revendications 1 à 10 sous la forme d'un kit de composants, comprenant des formes posologiques unitaires avec le composé de formule (I), ou un sel pharmaceutiquement acceptable de celui-ci, et le tramétinib, ou un sel pharmaceutiquement acceptable de celui-ci, ou un solvate de celui-ci, dans des formulations séparées et/ou dans des formulations combinées, pour administration simultanée, concomitante ou séquentielle, notamment pour qu'elles soient conjointement efficaces ; ledit kit comprenant facultativement en outre des instructions d'utilisation.

12. Inhibiteur de Raf qui est un composé de formule (I) tel que défini dans la revendication 1, ou un sel pharmaceutiquement acceptable de celui-ci, pour une utilisation dans une thérapie d'association avec le tramétinib, ou un sel ou solvate pharmaceutiquement acceptable de celui-ci, dans le traitement d'un sarcome (tel qu'un sarcome porteur d'une mutation de RAS), en particulier un rhabdomyosarcome ou plus particulièrement un rhabdomyosarcome embryonnaire (ERMS).

13. Tramétinib, ou un sel ou solvate pharmaceutiquement acceptable de celui-ci, pour une utilisation dans une thérapie d'association avec un inhibiteur de Raf qui est un composé de formule (I) tel que défini dans la revendication 1, ou un sel pharmaceutiquement acceptable de celui-ci, dans le traitement d'un sarcome (tel qu'un sarcome porteur d'une mutation de RAS), en particulier un rhabdomyosarcome ou plus particulièrement un rhabdomyosarcome embryonnaire (ERMS).
